# FASCICULE DE BREVET EUROPEEN

(11) **EP 0 705 072 B1**
(45) Date de publication et mention de la délivrance du brevet: **26.06.2002**
(21) Numéro de dépôt: 95915712.4
(22) Date de dépôt: 19.04.1995
(51) Int. Cl.: A61B 5/22, A61B 8/00

(54) **TABLE D'EXAMEN ECHOCARDIOGRAPHIQUE**
UNTERSUCHUNGSTISCH FÜR ECHOKARDIOGRAPHIE
ECHOCARDIOGRAPHIC EXAMINATION TABLE

(30) Priorité: 20.04.1994 BE 9400406
(43) Date de publication de la demande: 10.04.1996
(73) Titulaire: Berthe, Christian, 4000 Liège (BE)
(72) Inventeur: Berthe, Christian, 4000 Liège (BE)
(74) Mandataire: Vanderperre, Robert
(86) Numéro de dépôt international: BE9500037
(87) Numéro de publication internationale: WO9528882

(56) Documents cités:
- EP-A- 0 251 656
- WO-A-93/23001
- DE-U- 9 103 737

## Description

La présente invention concerne une table destinée à être utilisée pour effectuer des examens échocardiographiques de repos et d'effort.

Les tables d'examen échocardiographique connues comportent une tablette horizontale montée sur un bâti muni de pieds à roulettes et un pédalier fixé sur la tablette et que le patient actionne par le mouvement des jambes pour les examens échocardiographiques d'effort. Le patient étant étendu sur le dos à l'horizontale, le mouvement des jambes doit être effectué dans des conditions inconfortables et il est nécessaire de prévoir des accessoires additionnels pour assurer l'immobilisation du thorax du patient.

On connaît par ailleurs une table pour permettre l'examen d'un patient humain, telle qu'indiquée dans le préambule de la revendication 1 annexée (v. DE-U-9103737).

Le but de l'invention est de proposer une table compacte ayant un encombrement minimum et permettant d'effectuer à la fois les examens au repos et les examens à l'effort dans des conditions de confort maximales pour le patient tout en assurant une grande stabilité du thorax du patient et ainsi une persistance de la qualité de l'image échocardiographique pendant l'effort.

Cet objectif est atteint suivant l'invention par une table d'échocardiographie telle que définie dans les revendications.

Plus particulièrement, la tablette est fixée sur un cadre monté sur des coulisseaux glissant le long d'un châssis porteur fixé au bâti, et la tablette est constituée d'un dossier et d'une assise attachés de manière à pouvoir pivoter autour d'un axe charnière fixé au cadre, le dossier et l'assise étant connectés à des moyens fixés au châssis porteur pour régler les inclinaisons du dossier et de l'assise. Le châssis porteur est monté à ses extrémités sur deux colonnes de support de manière à pouvoir basculer autour d'un axe de pivotement longitudinal. Le pédalier est attaché au châssis porteur précité et il peut être relié à un dispositif d'entraînement pour en régler la hauteur par rapport au châssis porteur précité.

Les particularités et avantages de l'invention apparaîtront plus clairement à la lecture de la description d'un exemple de mode de réalisation représenté dans les dessins joints.

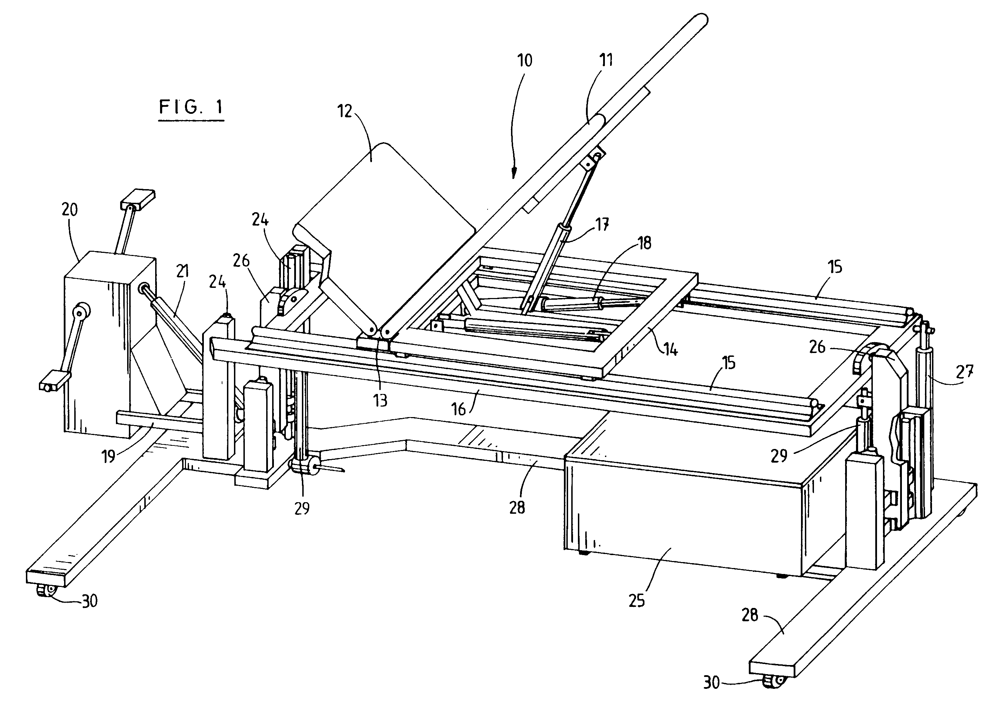

La figure 1 est une vue en perspective d'une table d'examen échocardiographique suivant l'invention, en position d'examen.

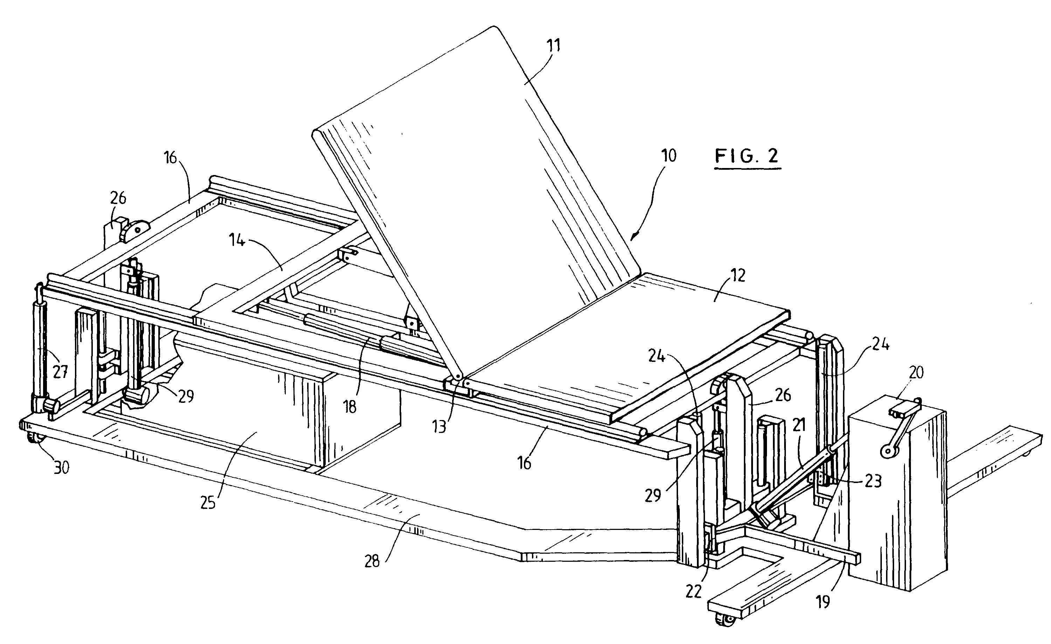

La figure 2 est une vue en perspective, avec arrachement partiel, de la table de la figure 1, en position d'attente.

La figure 3 est une vue en perspective de la table représentée en figure 1, avec le châssis montré en position basculée vers la gauche.

Comme on le voit sur les dessins, la tablette 10 de la table d'examen suivant l'invention comprend un dossier 11 et une assise 12 solidarisés entre eux par une charnière 13 qui permet d'incliner séparément le dossier 11 d'une part et l'assise 12 d'autre part. La charnière 13 est fixée à un cadre métallique 14 monté sur des coulisseaux glissant le long de deux rails parallèles 15 fixés sur les côtés longitudinaux d'un châssis porteur 16. Le glissement du cadre 14 le long des rails 15 permet le déplacement antéro-postérieur du dossier 11 et de l'assise 12 par rapport au châssis. Sur le cadre 14 sont fixés des vérins 17 et 18 commandant les inclinaisons individuelles du dossier 11 et de l'assise 12 respectivement.

Au châssis porteur 16 est également attachée une fourche 19 portant un pédalier 20 qui se trouve ainsi entraîné avec le châssis dans son mouvement de basculement latéral. Le pédalier 20 est connecté à un vérin 21 permettant un mouvement ascensionnel indépendant et réglable du pédalier par rapport au châssis 16. Pendant le mouvement ascensionnel du pédalier, les extrémités 22 et 23 de la fourche 19 coulissent le long des guides 24. Le pédalier est avantageusement relié à un dispositif électronique 25 agencé pour réaliser des programmes de charges progressives de manière à graduer les efforts à fournir par le patient.

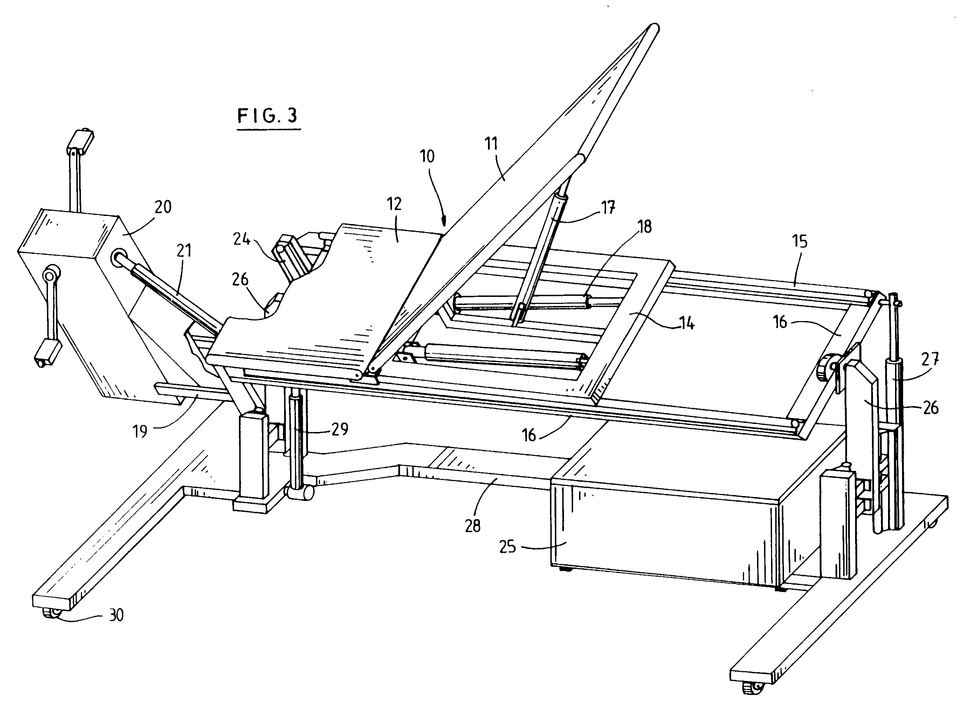

Le châssis porteur 16 est constitué de poutrelles longitudinales reliées à leurs extrémités par des poutrelles transversales. Celles-ci sont fixées en leur milieu à deux colonnes de support 26 de manière à pouvoir basculer autour d'un axe de pivotement longitudinal 100, le châssis entraînant le dossier 11 et l'assise 12 dans un mouvement de basculement latéral autour du grand axe antéro-postérieur de la table (voir figure 3). Le basculement du châssis autour de l'axe antéro-postérieur précité est commandé par un vérin 27 connecté entre le châssis 16 et le bâti 28 de la table.

Les colonnes de support 26 sont fixées au bâti 28 monté sur roulettes 30. Les colonnes 26 sont munies de vérins 29 permettant le mouvement ascensionnel du châssis 16 et de l'ensemble qu'il porte par rapport au bâti de la table.

La table suivant l'invention permet de réaliser des examens échocardiographiques à l'effort dans des conditions de confort maximales pour le patient grâce au fait que celui-ci peut se trouver en position semi-couchée. Le dossier 11 peut avantageusement être incliné suivant un angle de 45° environ par rapport au plan horizontal et l'assise 12 peut avantageusement être inclinée suivant un angle de 30° environ. En outre, la position du patient par rapport au pédalier s'ajuste de façon précise et rapide par le glissement horizontal antéro-postérieur du dossier et de l'assise. Le mouvement ascensionnel du pédalier positionne parfaitement celui-ci en hauteur afin de réduire au maximum la fatigue des membres inférieurs du patient au cours de l'effort.

Cette position confortable pour le patient permet la réalisation d'un effort maximal tout en évitant la fatigue rapide de ses membres inférieurs et les mouvements parasites de son thorax. L'image échocardiographique reste dès lors d'excellente qualité et parfaitement stable. Grâce au fait que la table d'examen permet au patient de rester tout à fait immobile, il serait possible d'y prévoir aisément un porte-sonde pour les mesures échocardiographiques.

Le réglage en hauteur du châssis et de l'ensemble qu'il porte rend possible d'effectuer des examens à gauche ou à droite du patient suivant les habitudes de l'examinateur.

La table d'examen suivant l'invention se complète d'un panneau de commande électrique (non représenté) réunissant les commandes de tous les déplacements et mouvements des éléments de la structure et les commandes des programmes d'efforts.

Lorsque le pédalier se trouve en position basse et l'assise en position horizontale, la table permet un examen échocardiographique de repos grâce à une courte rallonge qui peut venir reposer sur le dessus du bloc de pédalier.

## Revendications

1. Table pour permettre l'examen d'un patient humain, comportant un châssis porteur (16), une tablette (10) pour porter un patient, et un pédalier (20) destiné à être actionné par le mouvement des jambes du patient reposant sur la tablette (10), la tablette (10) étant constituée d'une partie postérieure (11) servant de dossier pour le patient et d'une partie antérieure (12) servant d'assise pour le patient, **caractérisée en ce que** la table comporte :
(i) un cadre (14) monté sur ledit châssis porteur (16) allongé de manière à pouvoir glisser longitudinalement sur ledit châssis suivant la direction antéro-postérieure de la table,
(ii) lesdites parties postérieure et antérieure de la tablette (10) étant reliées entre elles par une charnière (13) fixée au cadre (14) de manière à pouvoir pivoter autour de cette charnière, ladite charnière (13) permettant d'incliner séparément ledit dossier (11) et ladite assise (12),
(iii) des moyens de positionnement (17, 18) fixés audit cadre (14) pour régler les inclinaisons individuelles du dossier (11) et de l'assise (12), et
(iv) le pédalier (20) précité attaché au châssis porteur (16) en une position adjacente à une extrémité dudit châssis porteur allongé (16).

2. Table suivant la revendication 1, **caractérisée en ce que** le châssis porteur (16) est monté à ses extrémités sur deux colonnes de support (22) fixées sur un bâti (28) de manière que ledit châssis porteur (16) soit capable de basculer latéralement autour de l'axe antéro-postérieur de la table.

3. Table d'examen suivant la revendication 2, **caractérisée en ce que** le châssis porteur (16) est connecté à un dispositif d'entraînement (26) pour en régler la hauteur par rapport au bâti.

4. Table d'examen suivant l'une quelconque des revendications précédentes, **caractérisée en ce que** le pédalier (20) est connecté à un dispositif d'entraînement (21) pour en régler la hauteur par rapport au châssis porteur précité.

5. Table d'examen suivant l'une quelconque des revendication précédentes, **caractérisée en ce que** le pédalier (20) est relié à un dispositif agencé pour programmer des charges progressives.

## Patentansprüche

1. Untersuchungstisch für Patienten bzw. Personen mit einem Traggestell (16), einer Tischplatte (10) zur Aufnahme des Patienten, einer Tretkurbelvorrichtung (20), dazu bestimmt, durch die Bewegung der Beine des Patienten, der auf der Tischplatte (10) liegt, betätigt zu werden, wobei die Tischplatte (10) gebildet wird durch einen hinteren Teil (11), der dem Patient als Rücklehne dient, und einem vorderen Teil (12), der dem Patienten als Sitz dient,
**dadurch gekennzeichnet, dass** dieser Tisch umfasst:
(i) einen Rahmen (14), so auf das gestreckte Traggestell (16) montiert, dass er in der Richtung der Längsmittelachse des Tisches auf dem genannten Traggestell gleiten kann,
(ii) ein Scharnier (13), befestigt am Rahmen (14), das den vorderen und den hinteren Teil der Tischplatte (10) verbindet, so dass diese um dieses Scharnier herum schwenken können, wobei das genannte Scharnier (13) ermöglicht, die genannte Rückenlehne (11) und den genannten Sitz (12) getrennt schräg zu stellen bzw. zu neigen,
(iii) Positionierungseinrichtungen (17, 18), befestigt an dem genannten Rahmen (14), um die individuellen Neigungen der Rückenlehne (11) und des Sitzes (12) einzustellen, und
(iv) die Befestigung der genannten Tretkurbelvorrichtung (20) am Traggestell (16) in einer Position, die sich in der Nähe eines Endes des genannten gestreckten Traggestells (16) befindet.

2. Untersuchungstisch nach Anspruch 1, **dadurch gekennzeichnet, dass** das Traggestell (16) mit seinen Enden an zwei Tragsäulen (26) festgemacht ist, die so an einem Untergestell (28) befestigt sind, dass der genannte Tragrahmen (16) seitlich gekippt werden kann um die Längsmittelachse des Tisches.

3. Untersuchungstisch nach Anspruch 2, **dadurch gekennzeichnet, dass** das Traggestell (16) zum Verstellen bzw. Einstellen seiner Höhe in Bezug auf das Untergestell (28) mit einer Mitnahmevorrichtung (29) verbunden ist.

4. Untersuchungstisch nach einem der vorhergehenden Ansprüche, **dadurch gekennzeichnet, dass** die Tretkurbelvorrichtung (20) mit einer Antriebsvorrichtung (21) verbunden ist, um ihre Höhe in Bezug auf den genannten Tragrahmen zu verstellen bzw. einzustellen.

5. Untersuchungstisch nach einem der vorhergehenden Ansprüche, **dadurch gekennzeichnet, dass** die Tretkurbelvorrichtung (20) mit einer Vorrichtung verbunden ist, die eingerichtet ist, um progressive Belastungen zu programmieren.

## Claims

1. A table for examining a human patient comprising a carrying chassis (16), a couch (10) for carrying a patient, and a crank gear (20) to be driven by the movement of the legs of the patient lying on the couch (10), the couch being comprised of a back portion (11) as back for the patient and of a seating portion (12) as seat for the patient, **characterized in that** the table comprises :
(i) a frame (14) mounted on said elongated carrying chassis (16) so as to be able to slide lengthwise on said chassis, according to the anterio-posterior axis of the table,
(ii) said back portion and seating portion of the couch (10) being reciprocally connected by a hinge (13) fixed to said frame (14) so as to be able to pivote around this hinge, said hinge (13) allowing that said back (11) and said seat (12) be inclined separately,
(iii) positioning means (17, 18) fixed to the frame (14) for individually adjust the inclinations of the back (11) and seat (12), and
(iv) said crank gear (20) attached to said carrying chassis (16) in a position adjacent one end of the elongated carrying chassis (16).

2. A table according to claim 1, **characterized in that** said carrying chassis (16) is mounted at its ends on two supporting columns (22) fastened on a support structure (28) so that the carrying chassis (16) be capable of laterally revolving around the antero-posterior axis of the table.

3. An examination table according to claim 2, **characterized in that** the carrying chassis (16) is connected to a driving means (26) for the control of its height in relation to the support structure.

4. An examination table according to anyone of the preceding claims, **characterized in that** the crank gear (20) is connected to a driving device (21) for the control of its height in relation to said carrying chassis.

5. An examination table according to anyone of the preceding claims, **characterized in that** the crank gear (20) is connected to means arranged for programming progressive loads.
